# EUROPEAN PATENT APPLICATION

(11) **EP 3 144 012 A1**
(43) Date of publication of application: **22.03.2017**
(21) Application number: 16188915.9
(22) Date of filing: 15.09.2016
(51) Int. Cl.: A61K 39/07, A61K 9/00, A61K 31/122, A61K 31/593, A61K 31/728

(54) **ORAL SUPPLEMENT**

(30) Priority: 17.09.2015 IT UB20153699
(71) Applicant: Fastmeditalia S.r.L., 20832 Desio (Monza Brianza) (IT)
(72) Inventor: PINNA, Marco, I-21051 ARCISATE (Varese) (IT); CAPPITELLI, Francesco, I-20124 MILANO (IT)
(74) Representative: Lunati & Mazzoni S.r.L.

(57) **Abstract**

An oral supplement is provided comprising hyaluronic acid, probiotics, and xanthan gum.

## Description

The present invention relates to an oral supplement preferably of the type described in the preamble of first claim.

In particular, the invention relates to a supplement to be used to treat oral ailments, namely relating to the gums, oral mucous membrane or other inner parts of the mouth.

As is known, the mouth may be subject to numerous ailments and problems such as gingivitis and aphthae.

Gingivitis is an inflammation of the gums causing redness, swelling and bleeding.

If neglected, gingivitis degenerates into periodontitis, a disease in which the inner layer of the gum and bone moves away from the teeth, forming pockets where bacteria and food debris is collected, causing the onset of infections.

Aphthae are ulcers of the oral mucous membrane where the lining epithelium is destroyed leaving the underlying connective tissue exposed. Such damage causes local inflammation which makes actions such as eating, talking and swallowing difficult and painful.

Today to resolve these problems toothpaste and, in some cases mouthwashes, are used containing broad spectrum antibacterial agents or disinfectants such as triclosan, chlorhexidine and/or antibiotics.

The prior art mentioned above has several significant drawbacks.

A first drawback given by the use of such antibacterial or disinfectants agents is to be identified in the fact that their use is not very effective. In fact, there are many cases in which despite constant use the onset of gingivitis, mouth ulcers or other similar problems of the oral cavity still occur.

Another drawback is the alleged cellular toxicity of some compounds (such as triclosan) which make the product unsuitable for use by children and which, in some countries, has led to the product being banned.

Another drawback is the fact that the broad spectrum of anti-microbial activity of known toothpastes and mouthwashes also causes the elimination of the useful microbial flora in the oral cavity.

In this situation the technical purpose of the present invention is to devise an oral supplement able to substantially overcome the drawbacks mentioned above.

Within the sphere of said technical purpose one important aim of the invention is to make an oral supplement particularly effective against gingivitis, aphthae and other ailments of the oral cavity.

Another important purpose of the invention is to devise a toxic-free oral supplement which can therefore also be used by children.

A further purpose of the invention is to have an oral supplement that does not affect the useful microbial flora in the oral cavity.

The technical purpose and specified aims are achieved by an oral supplement as claimed in appended claim 1.

Herein, the measures, values, shapes and geometric references (such as perpendicularity and parallelism), when used with words like "about" or other similar terms such as "approximately" or "substantially", are to be understood as except for measurement errors or inaccuracies due to production and/or manufacturing errors and, above all, except for a slight divergence from the value, measure, shape or geometric reference which it is associated with. For example, said terms, if associated with a value, preferably indicate a divergence of not more than 10% of said value.

The oral supplement according to the invention is suitable to use for the treatment and disinfection of the oral cavity so as to prevent/cure aphthae, bad breath, gingivitis or other ailments of the oral cavity. It is preferably in the form of a liquid substance, more preferably a concentrated liquid substance which can be diluted in water.

It comprises hyaluronic acid, probiotics and at least one vitamin.

In particular, the oral supplement comprises low-molecular-weight hyaluronic acid, which is characterised by a molecular weight, substantially less than 1000 Dalton.

The content of hyaluronic acid in the oral supplement is practically below 5% in detail, substantially between 1% and 3% and preferably substantially equal to 2%. It should be noted that the percentage content of hyaluronic acid and any other component of the food supplement is defined according to the ratio of the weight of said component and the total weight of the food supplement.

The oral supplement also contains probiotics comprising, preferably exclusively, Lactobacillaceae and, in particular, Lactobacillaceae salivarius.

The content of Lactobacillaceae, preferably salivarius, is substantially less than 4%, in detail substantially between 1% and 2% and, in more detail, substantially equal to 1.3% (preferably 1.32%).

In addition to the said substances, the oral supplement may include a polysaccharide preferably identifiable in xanthan gum, also known as xanthan and commercially known as E415.

The polysaccharide content, namely the xanthan gum, is substantially between 5% and 20%, in particular, between 10% and 15% and, more specifically, substantially equal to 12%.

To summarise, in a preferred, non-limiting embodiment the oral supplement substantially comprises 2% of hyaluronic acid, 1.32% of Lactobacillaceae salivarius and 12% of xanthan gum.

Optionally, the oral supplement may comprise at least one vitamin appropriately chosen from at least one out of vitamin C, vitamin D3 and vitamin K2. Preferably, the oral supplement comprises several vitamins and, to be precise, comprises only vitamins C, D3 and K2.

The content of vitamin C is substantially between 8% and 1%, to be precise, between 4% and 2% and, more specifically, substantially equal to 3.2%.

The content of vitamin D3 is practically less than 0.5%, preferably substantially between 0.01% and 0.15%; and, more preferably, substantially equal to 0.09%.

The content of vitamin K2 is substantially lower than 3%, in detail, substantially between 0.5% and 1.5%; and, in more detail, substantially equal to 0.75%.

To summarise, in a preferred, non-limiting embodiment, the oral supplement substantially comprises 2% of hyaluronic acid, 1.32% of Lactobacillaceae salivarius, 3.2% of vitamin C, 0.09% of vitamin D3, 0.75% of vitamin K2 and 12% of xanthan gum.

In addition, the oral supplement may include at least one out of a colouring, flavouring and sweetener. Preferably, it comprises at least one colouring, one flavouring and one sweetener.

The colouring is chosen from among beta carotene, preferably 1% (i.e. with a concentration of colouring principle substantially equal to 1% (commercially known as E160A)); and blue carmine colouring (E132).

Preferably, the oral supplement comprises both the 1% beta carotene colouring and the blue carmine colouring.

The content of beta carotene colouring, preferably 1 %, is substantially less than 0.5%, in particular between 0.15% and 0.01% and, more particularly, practically equal to 0.08%.

The content of blue carmine colouring is substantially less than 0.4%, in particular 0.1% and, more particularly, practically equal to 0.02%.

The content of flavouring is substantially lower than 5%, in detail, substantially between 1% and 2%; and, in more detail, substantially equal to 1.6%.

The sweetener is preferably xylitol.

Its content is practically more than 50%, in detail substantially between 70% and 85% and, in more detail, substantially equal to 79% (preferably 78.94%).

In a preferred, non-limiting embodiment the oral supplement is substantially composed of 2% of hyaluronic acid, 1.32% of Lactobacillaceae salivarius, 3.2% of vitamin C, 0.09% of vitamin D3, 0.75% of vitamin K2, 12% of xanthan gum, 0.08% of beta carotene colouring, 0.02% of blue carmine colouring, 12% of xanthan gum, 1.6% of flavouring and 78.94% of xylitol.

Consequently, for example, in 2500 mg of oral supplement there are substantially 50 mg of hyaluronic acid, 33 mg of Lactobacillaceae salivarius, 80 mg of vitamin C, 2.22 mg of vitamin D3, 18.75 mg of vitamin K2, 300 mg of xanthan gum, 2 mg of 1% beta carotene colouring, 0.5 mg of blue carmine colouring and 1973.53 mg of xylitol.

The use of the oral supplement, described above in a structural sense, is as follows.

Initially, the user pours about 2.5 g of oral supplement into water and, in particular, into 50 ml of still water at room temperature.

It may be noted how the xanthan gum, when the oral supplement is dissolved in water, hydrates forming a mucoadhesive gel containing the various components of the oral supplement.

After the user has poured the content into the water, he drinks the water and supplement without swallowing it, letting said mixture sit in his mouth for about a couple of minutes and then swallowing it.

The invention achieves important advantages.

A first important advantage is the fact that the innovative combination of hyaluronic acid, probiotics and one or more vitamins assures the oral supplement high efficacy against virtually all infections of the oral cavity and, in particular, against aphthae and gingivitis.

In fact, the synergic effect given by the simultaneous presence of hyaluronic acid, probiotics and one or more vitamins gives the supplement a complete disinfectant and preventive action of the oral cavity.

Such action is determined in the first instance by the protective action of the gums given by the hyaluronic acid.

In addition, the use of low molecular weight hyaluronic acid gives said acid molecular properties very similar to the physiological properties of the oral mucous membrane. As a result, the low molecular weight hyaluronic acid is able to provide immediate and lasting relief from pain, promote tissue reconstruction and reduce the number and duration of damaging apthae.

The effect of the hyaluronic acid on the gums is assisted by the probiotics and, in particular by the Lactobacillaceae salivarius which helps to reduce gum inflammation.

This coadjuvant action of the hyaluronic acid in the treatment of gingivitis and similar ailments is determined by the presence at least one vitamin in the oral supplement.

Specifically, the vitamin C contributes to normal collagen formation required for the normal function of the gums, vitamin D helps to keep bones normal facilitating the absorption and use of calcium and phosphorus, while vitamin K2 promotes the uptake of free calcium circulating in the bloodstream, thus promoting bone deposition and helping the mineralization of teeth.

Lastly, the presence of a muco-adhesive such as xanthan gum improves the action of the oral supplement and, in particular, of the hyaluronic acid facilitating the adhesion of the supplement to the walls of the oral cavity and thus prolonging its contact time with said walls.

An important advantage is the fact that the xanthan gum, when the supplement is dissolved in water, hydrates, forming a gel which, being characterized by a mucoadhesive action, favours the adhesion of the oral supplement to the inner walls of the oral cavity and hence the effectiveness of local action of the oral supplement.

An additional benefit given by the probiotics and, particularly, by the Lactobacillaceae salivarius is to be identified in the fact that, being able to reside in the mouth, they adhere to the saliva and tissue of the mouth thereby constituting a natural and beneficial antibacterial agent against the pathogenic bacteria responsible for periodontal disease, caries and bad breath.

Another advantage of no less importance is the fact that the oral supplement, unlike mouthwashes or other similar products currently on the market, is composed exclusively of non-toxic components without side effects thereby resulting suitable for taking and swallowing without any risk, even by very young children.

Variations may be made to the invention without departing from the scope of the inventive concept described in the independent claims and in the relative technical equivalents. In said sphere all the details may be replaced with equivalent elements and the materials, shapes and dimensions may be as desired.

## Claims

1. Oral supplement **characterized by** comprising:
- hyaluronic acid;
- probiotics; and
- Xanthan gum.

2. Oral supplement according to claim 1, wherein said hyaluronic acid has a low molecular weight.

3. Oral supplement according to claim 1, wherein the content of said hyaluronic acid is substantially between 1% and 3%.

4. Oral supplement according to claim 1, wherein said probiotics comprise Lactobacillaceae salivarius.

5. Oral supplement according to claim 4, wherein the content of said Lactobacillaceae salivarius is substantially between 1% and 2%.

6. Oral supplement according to claim 1, wherein the content of said Xanthan gum is substantially between 10% and 15%.

7. Oral supplement according to claim 1, comprising at least one vitamin chosen from at least one out of vitamin C, vitamin D3 and vitamin K2.

8. Oral supplement according to claim 7, comprising a plurality of said at least one vitamin and wherein said vitamins comprise said vitamin C, said vitamin D3 and said vitamin K2.

9. Oral supplement according to claim 8, wherein the content of said vitamin C is substantially between 2% and 4%; wherein the content of said vitamin D3 is substantially between 0.01% and 0.15%; and wherein the content of said vitamin K2 is substantially between 0.5% and 1.5%.

10. Oral supplement according to claim 1, wherein said content of said hyaluronic acid content is substantially equal to 2%; wherein said content of said Lactobacillaceae salivarius is substantially equal to 1.32%; wherein said content of said Xanthan gum is substantially equal to 12%; wherein said content of said vitamin C is substantially equal to 3.2%; wherein said content of said vitamin D3 is substantially equal to 0.09%; and wherein said content of said vitamin K2 is substantially equal to 0.75%.
